# EUROPEAN PATENT APPLICATION

(11) **EP 2 535 045 A1**
(43) Date of publication of application: **19.12.2012**
(21) Application number: 11382197.9
(22) Date of filing: 15.06.2011
(51) Int. Cl.: A61K 9/50, A61K 31/4439

(54) **Pharmaceutical composition of lansoprazole**

(71) Applicant: Laboratorios del. Dr. Esteve, S.A., 08041 Barcelona (ES)
(72) Inventor: Encina García, Gregorio Jose, E - 08025 Barcelona (ES); Xu, Zhengguo, E - 08173 Sant Cugat del Vallés - Barcelona (ES)
(74) Representative: ABG Patentes, S.L.

(57) **Abstract**

The invention refers to an oral solid pharmaceutical composition comprising a mixture of: (a) pellets comprising lansoprazole or a pharmaceutically acceptable salt thereof being free of alkaline-reacting compounds and (b) pellets comprising lansoprazole or a pharmaceutically acceptable salt thereof together with alkaline-reacting compounds.

## Description

### FIELD OF THE INVENTION

The present invention refers to an oral solid pharmaceutical composition comprising a mixture of pellets comprising lansoprazole.

### BACKGROUND

Lansoprazole or 2-[[[3-methyl-4-(2,2,2-trifluoroethoxy)-2-pyridyl] methyl] sulfinyl] benzimidazole (marketed in Europe under the trademark PREVACID®), is a substituted benzimidazole that inhibits gastric acid secretion. The empirical formula of lansoprazole is C₁₆H₁₄F₃N₃O₂S and the compound has a molecular weight of 369.37. The structural formula of lansoprazole is (I):

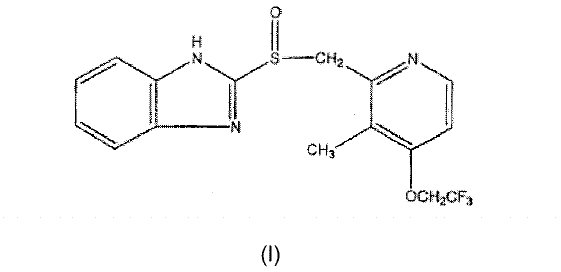

Lansoprazole belongs to a class of antisecretory compounds called proton pump inhibitors ("PPIs") that are prescribed, *inter alia,* for short-term treatment of active duodenal ulcers, gastric ulcers, gastroesophageal reflux disease (GERD), severe erosive esophagitis, poorly responsive systematic GERD, and pathological hypersecretory conditions such as Zollinger Ellison syndrome.

PPIs are, however, susceptible to degradation/transformation in acidic and neutral media. The degradation is catalyzed by acidic compounds and is stabilized in mixtures with alkaline compounds. The stability of the active substances is also affected by moisture, heat, organic solvents and to some degree by light.

With respect to the stability properties of the active substances, it is known that an oral dosage form should be protected from contact with the acidic gastric juice or comprise suitable components to neutralise the acidic gastric juice so that the active substance can be transferred in intact form to that part of the gastrointestinal tract where pH is near neutral and where rapid absorption can occur.

A pharmaceutical oral dosage form of PPIs is best protected from contact with acidic gastric juice by an enteric coating layer. For oral administration, commonly used solid dosage forms are capsules and tablets comprising a multitude of enteric coated pellets of the active ingredient. For instance, US 5,026,560 describes suitable enteric coated lansoprazole preparation. Said preparation contains an inert core which is coated with a layer comprising lansoprazole together with an alkaline reacting material, then a separating layer is applied and finally, it is coated with an enteric coating layer.

A further problem exists with PPIs. According to their prescribing information, these drugs, particularly its pharmacokinetic properties, are affected by the presence of food in the stomach at the time of dosing or shortly thereafter. Food can alter bioavailability by various means including: delay gastric emptying, stimulate bile flow, change gastrointestinal pH, increase visceral blood flow, change luminal metabolism of a drug substance, physically or chemically interact with a dosage form or a drug substance.

In order to provide a PPI-containing dosage form in which the drug substance exhibits a predictable bioavailability, EP 1 681 052 A1 provides a pharmaceutical dosage form comprising a solid core with the pharmaceutical active and a disintegrant, a swellable coating surrounding the core; and an enteric coating surrounding the swellable coating; wherein the enteric coating comprises between 3% and 7% of the weight of the dosage form; and wherein the dosage form provides equivalent bioavailability whether administered to a fasted or fed subject.

There is still a need for further PPI-containing dosage forms in which the PPI exhibits a predictable bioavailability when compared with the reference product, whether or not the dosage form is administered with food and in which the stability properties of the active substance are assured.

### BRIEF DESCRIPTION OF THE INVENTION

The inventors of the present invention have surprisingly found that an oral solid pharmaceutical composition comprising a mixture of two different kinds of lansoprazole pellets show for the active ingredient a predictable bioavailability in accordance with the reference product. Furthermore said pharmaceutical composition is chemically and physically stable and has a good *in vitro* dissolution rate.

In particular, one aspect of the present invention relates to an oral solid pharmaceutical composition (also referred herein simply as composition of the invention) comprising a mixture of:
(a) pellets comprising lansoprazole or a pharmaceutically acceptable salt thereof being free of alkaline-reacting compounds, and
(b) pellets comprising lansoprazole or a pharmaceutically acceptable salt thereof together with at least one alkaline-reacting compound,
in which the weight percentage of pellets (a) is between 25% (w/w) and 35% (w/w), preferably between 27% (w/w) and 33% (w/w) and more preferably between 29% (w/w) and 31% (w/w) with respect to the total weight of the pellets comprised in the pharmaceutical composition.

According to another aspect, the invention relates to a capsule, in particular a gelatine capsule, comprising the pharmaceutical composition of the invention.

In a second aspect, the present invention relates to the pharmaceutical composition as defined herein for use in the treatment and/or prophylaxis of a gastrointestinal disorder.

In a further aspect, the present invention relates to the use of the pharmaceutical composition as defined herein for the preparation of a medicament for the treatment and/or prophylaxis of a gastrointestinal disorder.

Another aspect of the present invention refers to a method for the treatment and/or prophylaxis of a gastrointestinal disorder, the method comprising administering to the subject in need of such a treatment or prophylaxis a therapeutically effective amount of the pharmaceutical composition described herein.

In one embodiment, the pharmaceutical composition of the invention is indicated for treatment and/or prophylaxis of ulcers of the stomach and duodenum, and NSAID-induced ulcers; gastroesophageal reflux disease (GERD) (also known as acid reflux disease); severe erosive esophagitis; poorly responsive systematic GERD; pathological hypersecretory conditions such as Zollinger-Ellison Syndrome; and adjunctive treatment of *Helicobacter pylori* infection, alongside antibiotics; or combinations of any of the above disorders.

These aspects and preferred embodiments thereof are additionally also defined hereinafter in the detailed description, as well as in the claims.

### DETAILED DESCRIPTION OF THE INVENTION

Lansoprazole, like other benzimidazole compounds of therapeutic interest, is labile in an acid medium, which creates a great number of problems when developing a pharmaceutical form meant for oral administration as said compound breaks down upon contact with the stomach contents, a strongly acidic medium. Further, the so-called "food effect" has been observed with lansoprazole since it is known that food can change the bioavailability of this active ingredient and consequently can influence the bioequivalence between the test and reference products. Thus, both its labile nature and food effects on bioequivalence are responsible for the variability in the intra- and inter-individual therapeutic response of lansoprazole and hence providing dosage forms that can be administered without regard to a patient's meal schedule, or even in the absence of a definite meal schedule, is a significant step toward reducing the variability of treatment efficacy.

### Pharmaceutical composition

Therefore, an object of this invention is to provide an oral solid pharmaceutical composition comprising lansoprazole which exhibits a predictable bioavailability in the presence or in the absence of food when compared with the reference product, excellent storage stability and optimum dissolution profile.

In particular, the composition of the invention comprises a mixture of:
(a) pellets comprising lansoprazole or a pharmaceutically acceptable salt thereof being free of alkaline-reacting compounds, and
(b) pellets comprising lansoprazole or a pharmaceutically acceptable salt thereof together with one or more alkaline-reacting compounds.

By the term "equivalence" or "bioequivalence" it is understood the following: two medicinal products containing the same active substance are considered bioequivalent if they are pharmaceutically equivalent or pharmaceutical alternatives and their bioavailabilities (rate and extent) after administration in the same molar dose lie within acceptable predefined limits (with a 90 percent confidence interval of ratio of the geometric mean between the test and the reference within 80.00 and 125.00 percent). These limits are set to ensure comparable *in vivo* performance, i.e. similarity in terms of safety and efficacy. See Guideline on the Investigation of Bioequivalence. European Medicines Agency. Doc. Ref.: CPMP/QWP/EWP/1401/98 Rev. 1.

By "pharmaceutically acceptable" such as in the recitation of a "pharmaceutically acceptable salt " or a "pharmaceutically acceptable excipient " is meant herein a material that is not biologically or otherwise undesirable, i.e., the material may be incorporated into a pharmaceutical composition administered to a patient without causing any undesirable biological effects or interacting in a deleterious manner with any of the other components of the composition in which it is contained.

The term "pharmaceutically acceptable salts" in the context of this invention means any salt that is tolerated physiologically (normally meaning that it is not toxic, particularly, as a result of the counter-ion) when used in an appropriate manner for a treatment, applied or used, particularly, in humans and/or mammals. Examples of pharmaceutically acceptable salts of the compound (I) include a salt with an inorganic base, a salt with an organic base, a salt with a basic amino acid and the like. Preferred examples of the salt with an inorganic base include alkali metal salts such as a sodium salt and a potassium salt; alkaline earth metal salts such as a calcium salt and a magnesium salt; an ammonium salt and the like.

The term "alkaline-reacting compound" is known by the skilled person and refers to a pharmaceutically acceptable substance which creates a "micro-pH" around the pellet of not less than pH=7, preferably not less than pH=8, when water is added in small amounts to said pellet. Such substances can be chosen among, but are not restricted to substances normally used in antacid preparations such as alkali (e.g. sodium, potassium), alkaline earth metal (e.g. calcium, magnesium) and aluminium salts of phosphoric acid, carbonic acid, bicarbonic acid, silicilic, citric acid or other suitable weak inorganic or organic acids; oxides or hydroxides of the above-mentioned cations; composite aluminium/magnesium substances, such as Al₂O₃·6MgO·CO₂·12H₂O, (Mg₆Al₂(OH)₁₆CO₃·4H₂O), MgO·Al₂O₃·2SiO₂·nH₂O or similar compounds; aluminium hydroxide/sodium bicarbonate coprecipitate or similar compounds; organic pH-buffering substances such as trihydroxymethylaminomethane or other similar pharmaceutically acceptable pH-buffering substances; suitable organic bases, including basic amino acids and salts thereof.

The inventors have found that low percentages of pellets containing alkaline-reacting compounds do not provide the desired bioequivalence. Concretely, it has been proved that an amount of pellets (a) between 25% (w/w) and 35% (w/w) with respect to the total weight of the pellets (a) and (b) comprised in the pharmaceutical composition is required to obtain a predictable bioavailability when compared with the reference product. Moreover, a composition with such an amount of pellets (a) has shown to be highly stable. According to a preferred embodiment, the weight percentage of the pellets (a) is between 25% (w/w) and 35% (w/w) with respect to the sum of pellets (a) and (b). According to a more preferred embodiment, pellets (a) may be present in an amount between 27% (w/w) and 33% (w/w). According to another preferred embodiment, pellets (a) may be present in an amount between 29% (w/w) and 31% (w/w) with respect to the total weight of the pellets comprised in the pharmaceutical composition. In a most specially preferred embodiment, the composition comprises about 31% of pellets (a) and about 69% of pellets (b).

As used herein, the term "about" means a slight variation of the value specified, preferably within 10 percent of the value specified. Nevertheless, the term "about" can mean a higher tolerance of variation depending on for instance the experimental technique used. Said variations of a specified value are understood by the skilled person and are within the context of the present invention. Further, to provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value. Unless otherwise stated, all amounts are expressed herein as percentage by weight.

According to a particular embodiment of the invention, the alkaline-reacting compound is selected from the group consisting of carbonates, bicarbonates, oxides and hydroxides of alkali and alkaline earth metals or a mixture thereof. Preferably, these alkali and alkaline earth metal salts are selected from sodium, potassium, magnesium and calcium salts, and more preferably, the alkaline-reacting compound is magnesium carbonate.

With the exception of the alkaline-reacting compound, pellets (a) and (b) may be very similar and comprise the same kind of components. Accordingly, for simplicity reasons and unless otherwise stated, the following description refers to both pellets (a) and (b).

The pellets of the pharmaceutical composition of the invention contain lansoprazole or a pharmaceutically acceptable salt thereof as active ingredient and advantageously are manufactured in multilayer form having a core-sheath structure.

The core of the pellet may comprise a monolithic particle comprising lansoprazole or its pharmaceutical salts or may be formed by an inert bead which is covered with a layer comprising the active ingredient. Preferentially, the core may be made from an inert bead which is covered by a layer comprising the active ingredient and optionally pharmaceutically acceptable excipients. The inert bead or core is inert with regard both to lansoprazole and to the other excipients in the pellet, and with regard to the patient who will ingest the pellet. Such inert bead is conventionally used in pharmaceutical techniques. The bead may be prepared from materials such as, e. g. starch, sucrose, microcrystalline cellulose, and the like. The size of the beads depends on the desired size of the pellet to be manufactured or further processed. Typically, the core represents from about 50% to 60% by weight of the pellet.

The amount of active ingredient (i.e. lansoprazole or a salt thereof) may vary for example from 5% to 15% with respect to the weight of the pellet. In particular, amounts of about 9% have been found to be effective. The layer containing the active ingredient (herein referred to as active layer; film coating 1 in the examples) may include excipients commonly used in pharmaceutical formulations that do not interact adversely with lansoprazole and its salts. In a preferred embodiment, the core of the pellets comprises an inert bead and said inert bead is covered by an active layer comprising lansoprazole, or a pharmaceutically acceptable salt, and pharmaceutically acceptable excipients, such as at least a binder. Examples of binders include, but are not limited to, hydroxypropylcellulose (or HPC), hydroxypropylmethylcellulose (or HPMC or hypromellose), hydroxypropyl cellulose, hydroxyethyl cellulose, sugars (such as sucrose, glucose and dextrose), or a combination thereof. In addition to the foregoing, the active layer can further contain other excipients such as disintegrants, colorants and the like. The following are examples of useful disintegrants: starches such as corn or potato starch, modified starches (such as sodium starch glycolate) and partially pregelatinized starches (such as Starch 1500); polyvinylpyrrolidones, including modified polyvinylpyrrolidones (such as crospovidone, polymerized under conditions that promote crosslinking); celluloses such as microcrystalline cellulose, modified celluloses (such as low substituted hydroxypropyl cellulose, croscarmellose sodium and calcium carboxymethyl cellulose). Example of colorants is titatium oxide. Additionally, pellets (b) preferably contain the alkaline-reacting compounds in the active layer.

Preferably, the pellets are individually enteric coated by one or more layers. Before applying enteric coating layer(s), said pellets are covered with one or, optionally, more separating layers comprising pharmaceutical excipients optionally including in pellets (b) alkaline compounds such as for instance pH-buffering compounds. This/these separating layer(s) separate(s) the active layer (or monolithic particle comprising lansoprazole) from the outer layer(s) being enteric coating layer(s).

The separating layer(s) can be applied to the active layer by coating or layering procedures in suitable equipments such as coating pan, coating granulator or in a fluidized bed apparatus using water and/or organic solvents for the coating process. As an alternative, the separating layer(s) can be applied to the active layer by using powder coating technique. The materials for separating layers are pharmaceutically acceptable excipients such as, for instance, binders (e.g. hydroxypropylcellulose or HPC, hydroxypropylmethylcellulose or HPMC or hypromellose, hydroxypropyl cellulose, hydroxyethyl cellulose), sugars (such as sucrose, glucose and dextrose), disintegrants (e.g. corn or potato starch, sodium starch glycolate, polyvinylpyrrolidones, including modified polyvinylpyrrolidones such as crospovidone, polymerized under conditions that promote crosslinking; celluloses such as microcrystalline cellulose, modified celluloses such as low substituted hydroxypropyl cellulose, croscarmellose sodium and calcium carboxymethyl cellulose), surfactants (e.g. polysorbates, sodium laurylsulfate), plasticizers (polyethylene glycol, acetylated monoglyceride, triacetin, castor oil and the like), lubricants (e.g. sodium stearylfumarate, magnesium stearate, hydrogenated vegetable oil, stearic acid, calcium stearate, glyceryl behenate, sodium lauryl sulphate, talc) colorants (e.g. titanium dioxide), used alone or in mixtures.

When the separating layer(s) is applied it may constitute a variable thickness. The maximum thickness of the optional separating layer(s) is normally only limited by processing conditions. The separating layer(s) may serve as a diffusion barrier and may act as a pH-buffering zone. The pH buffering properties of the separating layer(s) can be further strengthened by introducing into the layer(s) substances chosen from a group of compounds usually used in antacid formulations such as those previously defined as "alkaline-reacting compounds": for instance, magnesium oxide, hydroxide or carbonate, aluminium or calcium hydroxide, carbonate or silicate; composite aluminium/magnesium compounds such as, for instance Al₂O₃·6MgO·CO₂·12H₂O, (Mg₆Al₂(OH)₁₆CO₃·4H₂O), MgO·Al₂O₃·2SiO₂·nH₂O, aluminium hydroxide/sodium bicarbonatecoprecipitate or similar compounds; or other pharmaceutically acceptable pH-buffering compounds such as, for instance the sodium, potassium, calcium, magnesium and aluminium salts of phosphoric, carbonic, citric or other suitable, weak, inorganic or organic acids; or suitable organic bases, including basic amino acids and salts thereof. Talc or other compounds may be added to increase the thickness of the layer(s) and thereby strengthen the diffusion barrier. The separating layer(s) may improve the chemical stability of the active ingredient and/or the physical properties of the pharmaceutical composition.

One or more enteric coating layers are applied onto the active layer or onto the active layer covered with separating layer(s) by using a suitable coating technique. The enteric coating layer material may be dispersed or dissolved in either water or in suitable organic solvents. As enteric coating layer polymers one or more, separately or in combination, of the following can be used; e.g. solutions or dispersions of methacrylic acid copolymers, polysorbates, cellulose acetate phthalate, hydroxypropyl methylcellulose phthalate, hydroxypropyl methylcellulose acetate succinate, polyvinyl acetate phthalate, cellulose acetate trimellitate, carboxymethylethylcellulose, shellac, or other suitable enteric coating layer polymer(s). Methacrylate-base coatings are preferred and several useful products are commercially available from Röhm GmbH & Co., Darmstadt, Germany under the trademark EUDRAGIT. EUDRAGIT L30 D-55 is especially preferred. EUDRAGIT L30 D-55 is an aqueous dispersion of a pH dependent polymer soluble at or above pH 5.5 for targeted delivery in the duodenum. EUDRAGIT L30 D-55 is a copolymer of methacrylic acid an ethyl acrylate in a 1:1 ratio and has the formula (C₅H₂O₂·C₄H₆O₂)ₓ.

The enteric coating layers may further contain pharmaceutically acceptable plasticizers to obtain the desired mechanical properties, such as flexibility and hardness of the enteric coating layers. Such plasticizers are for instance, but not restricted to, triacetin, citric acid esters, phthalic acid esters, dibutyl sebacate, cetyl alcohol, polyethylene glycols, polysorbates or other plasticizers. Anti-tacking and anti-static agents, such as for instance magnesium stearate, titanium dioxide, talc and other additives may also be included into the enteric coating layer(s), being titanium dioxide and/or talc preferred.

According to a preferred embodiment, the active layer is covered by exactly two layers: a separating layer and an enteric layer over the separating layer (respectively, film coatings 2 and 3 in the examples).

In a more preferred embodiment, both pellets (a) and (b) comprise:
(1) an inert core;
(2) an active layer, deposited over said inert core (1), comprising lansoprazole or a pharmaceutically acceptable salt thereof, at least one binder preferably selected from hydroxypropylcellulose, hydroxypropylmethylcellulose and sucrose or a combination thereof, and, in the case of pellets (b) additionally at least one alkaline-reacting compound;
(3) a separating layer; and
(4) an enteric layer over the separating layer (3).

According to a preferred embodiment, the composition of the pellets (a) comprises:
(1) an inert core;
(2) an active layer, deposited over said inert core (1), comprising lansoprazole or a pharmaceutically acceptable salt thereof, and hydroxypropylmethylcellulose;
(3) a separating layer comprising hydroxypropylmethylcellulose; and
(4) an enteric layer over the separating layer (3).

According to a most preferred embodiment, the enteric layer of the pellets (a) comprises Eudragit L30 D-55, talc and triethyl citrate.

According to another preferred embodiment, the composition of the pellets (b) comprises:
(1) an inert core;
(2) an active layer, deposited over said inert core (1), comprising lansoprazole or a pharmaceutically acceptable salt thereof, hydroxypropylcellulose, sucrose, corn starch, low substituted hydroxypropylcellulose, and magnesium carbonate;
(3) a separating layer comprising hydroxypropylcellulose, sucrose, corn starch, low substituted hydroxypropylcellulose; and
(4) an enteric layer over the separating layer (3).

According to a most preferred embodiment, the enteric layer of the pellets (b) comprises Eudragit L30D-55, talc, polyethylene glycol 6000, titanium oxide and polysorbate 80.

Further, the pharmaceutical composition of the invention may be in the form of a compressed dosage form such as a tablet, or alternatively, the pellets may be filled into capsules or sachets. Preferably, the pharmaceutical composition of the invention is provided in form of capsules e.g., soft or hard gelatin and non-gelatin capsules. Non-gelatin capsules are for instance those made up of plant polysaccharides or their derivatives (like carrageenans and modified forms of starch and cellulose).

In a preferred embodiment, the capsule further comprises a lubricant, which is preferably selected from sodium stearylfumarate, magnesium stearate, hydrogenated vegetable oil, stearic acid, calcium stearate, glyceryl behenate, sodium lauryl sulphate and talc, the latter being preferred. The combination of above-mentioned lubricants can also be used. Typically the lubricant(s) is present in less than 1% by weight with respect to the sum of the weights of pellets (a), (b) and lubricant(s).

### Process

The process for the manufacture of the pharmaceutical composition represents a further aspect of the invention. The mentioned formulations will be prepared using standard methods such as those described or referred to in the European and US Pharmacopoeias and similar reference texts. The pharmaceutical processes can preferably be completely water-based and there are different descriptions given in the accompanying examples below.

In a particular embodiment, the process for preparing the composition of the invention comprises the steps of:
i) preparing independently pellets (a) and (b) by:
   - coating the inert core with an active layer comprising lansoprazole or a pharmaceutically acceptable salt thereof, at least one binder preferably selected from hydroxypropylcellulose, hydroxypropylmethylcellulose and sucrose or a combination thereof, and, in the case of pellets (b) additionally at least one alkaline-reacting compound;
   - optionally, coating with one or more layers such as a separating layer and an enteric layer.
ii) mixing the required amount of pellets (a) and (b) and optionally at least one pharmaceutical acceptable excipient such as a lubricant; and
iii) optionally, encapsulating the mixture to obtain capsules.

### Use of the pharmaceutical composition

The pharmaceutical composition according to the invention is especially advantageous in reducing gastric acid secretion.

In a further aspect, the present invention relates to a method of treating a gastrointestinal disorder. The method involves the step of administering to a patient in need of such a treatment (notably a human) a therapeutically effective amount of the pharmaceutical composition described herein. Gastrointestinal disorders that can be treated using the hereinbefore described method include, but are not limited to, ulcers of the stomach and duodenum, and NSAID-induced ulcers; gastroesophageal reflux disease (GERD) (also known as acid reflux disease); severe erosive esophagitis; poorly responsive systematic GERD; pathological hypersecretory conditions such as Zollinger-Ellison Syndrome; and adjunctive treatment of *Helicobacter pylori* infection, alongside antibiotics; or combinations of any of the above disorders.

In one embodiment, the pharmaceutical composition of the invention is specifically indicated for short-term treatment of active duodenal ulcer, H. pylori eradication to reduce the risk of duodenal ulcer recurrence, maintenance of healed duodenal ulcers, short-term treatment of active benign gastric ulcer, healing of NSAID-associated gastric ulcer, risk reduction of NSAID-associated gastric ulcer, short-term treatment gastroesophageal reflux disease (GERD), maintenance of healing of erosive esophagitis (EE), long-term treatment pathological hypersecretory conditions Including Zollinger-Ellison Syndrome (ZES).

Generally an effective administered amount of lansoprazole will depend on the severity of the disorder being treated and the weight of the sufferer. However, the pharmaceutical composition of the invention will typically be administered once or more times a day for example 1, 2, 3 or 4 times daily, with typical total daily doses of lansoprazole in the range of from 1 to 400 mg, such as 15 or 30 mg.

The following examples are merely illustrative of certain embodiments of the invention and cannot be considered as restricting it in any way.

### Examples

### EXAMPLE 1 - CAPSULE FORMULATION

### - Pellets (a): Composition and process of manufacture

| **Pellet** | **Component** | **Unit formula** | **%** |
|---|---|---|---|
| **Core** | Inert beads | 206,00 | 61,05 |
| **FC 1** | Lansoprazole | 30,00 | 8,89 |
| | Hypromellose+water | 22,92 | 6,79 |
| **FC 2** | Hypromellose | 33,53 | 9,94 |
| | Titanium dioxide+water | 4,47 | 1,32 |
| **FC 3** | Eudragit L 30D-55 | 32,18 | 9,54 |
| | Talc | 3,47 | 1,03 |
| | Triethyl citrate+water | 4,85 | 1,44 |

| | | | |
|---|---|---|---|
| FC = film coating; FC 1 = active layer; FC 2 = separating layer; FC 3 = enteric layer | | | |

1. FC 1 was prepared as follows: First, Lansoprazole was dispersed into purified water by constant stirring. In parallel, hypromellose was dispersed into purified water by stirring. Next, the resulting hypromellose suspension is added to the lansoprazole suspension while stirring.
2. Inert beads were coated by pulverising the suspension FC 1 using in a fluid bed drying granulator.
3. FC 2 was prepared by dispersing hypromellose and titanium dioxide into purified water by constant stirring.
4. FC 1 coated pellets were coated by pulverising the suspension FC 2. Then, the FC 2 pellets were dried.
5. FC 3 was prepared as follows: First, triethycitrate was dispersed by stirring into purified water. The resulting suspension is added to a methacrylic acid copolymer suspension. Then, Talc was dispersed by stirring into purified water and the resulting suspension was added to the suspension of triethylcitrate and methacrylic acid copolymer. The final FC 3 suspension was homogenised and maintained under constant stirring until required.
6. The FC 2 pellets of step 4 were loaded in the fluid bed drying granulator and coated by pulverising the suspension FC 3.
7. The FC 3 coated pellets were dried and sieved.

### - Pellets (b): Composition and process of manufacture

| **Pellet** | **Component** | **Unit formula** | **%** |
|---|---|---|---|
| **Core** | Inert beads | 159,41 | 49,80 |
| **FC 1** | Lansoprazole | 30,00 | 9,37 |
| | Magnesium Carbonate | 17,94 | 5,61 |
| | Corn Starch | 3,40 | 1,06 |
| | Sucrose | 15,93 | 4,98 |
| | Low substituted HPC | 5,98 | 1,87 |
| | Hydroxypropylcellulose+water | 23,49 | 7,34 |
| **FC 2** | Sucrose | 9,63 | 3,01 |
| | Corn Starch | 9,63 | 3,01 |
| | Low substituted HPC | 9,60 | 3,00 |
| | Hydroxypropylcellulose+water | 3,58 | 1,12 |
| **FC 3** | Eudragit L30D-55 | 20,16 | 6,30 |
| | Talc | 6,38 | 1,99 |
| | Polyethylene glycol 6000 | 1,99 | 0,62 |
| | Titanium oxide | 1,99 | 0,62 |
| | Polysorbate 80+water | 0,96 | 0,30 |

| | | | |
|---|---|---|---|
| FC = film coating; FC 1 = active layer; FC 2 = separating layer; FC 3 = enteric layer | | | |

1. FC 1 was prepared as follows: first, Lansoprazole, magnesium carbonate, corn starch, sucrose, L-HPC and HPC were dispersed into purified water by constant stirring
2. Inert beads were coated by pulverising the suspension FC 1 using a fluid bed drying granulator. Alternatively, FC 1 could be divided in two fractions which are subsequently applied onto the sugar beads.
3. FC 2 was prepared by corn starch, sucrose, L-HPC and HPC into purified water by constant stirring.
4. FC 1 pellets of step 2 were coated by pulverising the suspension FC 2. Then, the resulting FC 2 pellets were dried.
5. FC 3 was prepared as follows: First, a dispersion of Eudragit L30D-55 in water is prepared and maintained under stirring. Second, PEG 6000 and polysorbate 80 were dispersed into purified water. Third, talc and Titanium dioxide were dispersed into purified water. Once prepared the three dispersions, the second and the third dispersions were subsequently added to the first (Eudragit) dispersion. The resulting FC 3 suspension was homogenised and maintained under constant stirring until required.
6. The FC 2 pellets of step 4 were loaded in the fluid bed drying granulator and coated by pulverising the FC 3 suspension..
7. The FC 3 coated pellets were dried and sieved.

### - Capsules and process of manufacture

| **Component** | **Unit formula** | **%** |
|---|---|---|
| Pellets (a) | 101,23 | 31,03 |
| Pellets (b) | 224,05 | 68,67 |
| Talc | 0,97 | 0,30 |

1. The required amounts of pellets (a), (b) and talc were mixed into a suitable mixer machine until the blend was homogeneous.
2. The resulting mixture of step 2 was encapsulated in order to obtain desired dose of lansoprazole.

### EXAMPLE 2 - STABILITY DATA OF FORMULATION OF EXAMPLE 1

**Table 1**

| **25°C / 60% RH** | | | **Months** | | |
|---|---|---|---|---|---|
| **Parameter** | **Shelf Life Specification** | | **0** | **3** | **6** |
| **Individual related substances** | **Known** | **OB89: NMT 0.2%** | < 0.05 % | < 0.05 % | < 0.05 % |
| | | **OB97-HA: NMT 0.4%** | < 0.05 % | < 0.05 % | 0.05 % |
| | | **OB97-HH: NMT 0.2%** | < 0.05 % | < 0.05 % | < 0.05 % |
| | | **OB96: NMT 0.2%** | < 0.05 % | < 0.05 % | 0.05 % |
| | **Higher unknown impurity** | **NMT 0.2%** | < 0.05 % | < 0.05 % | < 0.05 % |
| **Total Impurities** | | **NMT 2.0 %** | < 0.05 % | < 0.05 % | 0.10 % |

| | | | | | |
|---|---|---|---|---|---|
| NMT not more than RH = Humidity relative | | | | | |

Specifications concerning the levels of impurities were calculated from the ICH Q6A guidelines: *SPECIFICATIONS: TEST PROCEDURES AND ACCEPTANCE CRITERIA FOR NEW DRUG SUBSTANCES AND NEW DRUG PRODUCTS: CHEMICAL SUBSTANCES,* wherein ICH stands for International Conference Harmonisation and Q refers to quality.

### CHEMICAL STRUCTURE OF IMPURITIES

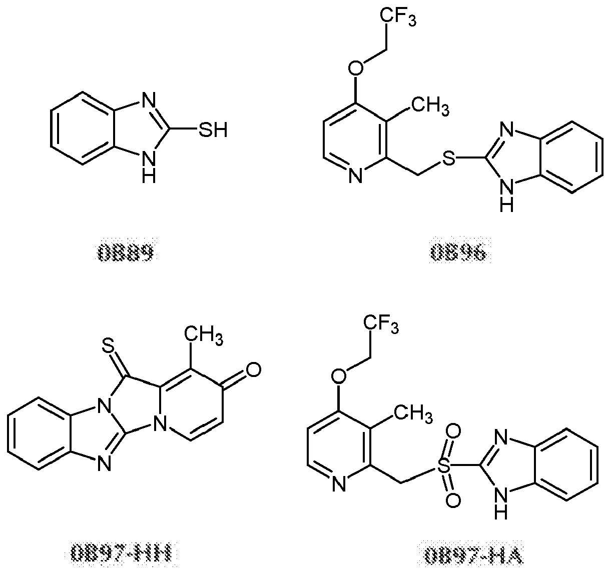

OB89 = 2-Mercaptobenzimidazole
0B97-HA = 2-[[[3-Methyl-4-(2,2,2- trifluoroethoxy)pyridin-2-yl]methyl]sulfonyl]benzimidazole
0B97-HH = 2,12-Dihydro-1-methyl-12-thioxopyrido[1',2':3,4]-imidazo[1,2-a]benzimidazol-2-one
OB96 = 2-[[[3-Methyl-4-(2,2,2-trifluoroethoxy) pyridin-2-yl]methyl]thio]benzimidazole

The stability results shown in Table 1 demonstrate that the formulation of Example 1 is stable in the conditions tested.

### EXAMPLE 3 - "IN VIVO" BIOEQUIVALENCE DATA OF EXAMPLE 1

An in vivo bioavailability test under fed conditions was conducted to compare the capsules of the present invention and PREVACID 30mg capsules. 59 subjects were completed at least two periods with test and reference crossed at least once in a 4-period, 2-sequence replicate crossover study and were included in statistical analysis evaluated in a two-way crossover study. The following table shows the results obtained:

**Table 2**

| **PARAMETER** | **GEOMETRIC LS MEANS** | | **RATIO** | **90% CONFIDENCE LIMITS** | |
|---|---|---|---|---|---|
| | **TEST** | **REFERENCE** | | **LOWER** | **UPPER** |
| **Cₘₐₓ** | 128.33 | 131.44 | 97.64 | 82.93 | 114.95 |
| **AUC_{T}** | 481.16 | 474.38 | 101.43 | 88.15 | 116.71 |
| **AUC_{∞}** | 499.96 | 488.37 | 102.37 | 89.34 | 117.31 |

The result shows that the ratios for the Cₘₐₓ, AUCₜ and AUC_{∞} are all within the 80-125% bioequivalence criteria, therefore the formulation of present invention is considered bioequivalent to the reference formulation.

### COMPARATIVE EXAMPLES

### Comparative Example 1

Two additional formulations were prepared with mixtures of pellets (a) and (b) outside the ranges of the invention. Qualitative and quantitative composition of pellets (a) and (b) are identical to that of Example 1. The manufacturing process of both pellets (a) and (b) is also the same as that of Example 1. However, differences between the three formulae (comparatives and Example 1) are in the content of pellets (a) measured as percentages (w/w).

Thus, comparative formulation I comprises 60% w/w of pellets (a) and comparative formulation II comprises 45% w/w of pellets (a).

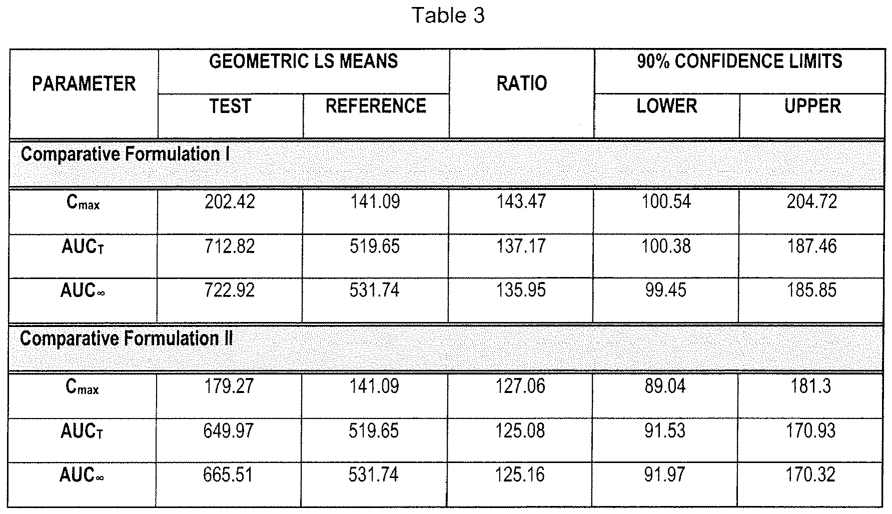

In this single center, randomized, single dose, laboratory-blinded, 3-period, 3-sequence, crossover comparative bioavailability study under fed conditions 22 subjects were included in the pharmacokinetic and statistical analysis. The results presented herein show that the criteria used to assess bioequivalence between each Test and Reference formulations were not fulfilled. The Tests to Reference ratio of geometric LSmeans and corresponding 90% confidence interval for the Cmax, AUC_{T} and AUC_{∞} were all outside the acceptance range of 80 to 125%.

## Claims

1. An oral solid pharmaceutical composition comprising a mixture of:
(a) pellets comprising lansoprazole or a pharmaceutically acceptable salt thereof being free of alkaline-reacting compounds and
(b) pellets comprising lansoprazole or a pharmaceutically acceptable salt thereof together with at least one alkaline-reacting compound
in which the weight percentage of pellets (a) is between 25% (w/w) and 35% (w/w) with respect to the total weight of the pellets (a) and (b) comprised in the pharmaceutical composition.

2. The pharmaceutical composition according to claim 1 in which the weight percentage of pellets (a) is between 27% and 33 % (w/w) with respect to the total weight of the pellets (a) and (b) comprised in the pharmaceutical composition.

3. The pharmaceutical composition according to claim 1 in which the weight percentage of pellets (a) is between 29% and 31 % (w/w) with respect to the total weight of the pellets (a) and (b) comprised in the pharmaceutical composition.

4. The pharmaceutical composition according to any of claims 1-3 wherein the alkaline-reacting compound is selected from carbonates, bicarbonates, oxides and hydroxides of alkali and alkaline earth metals or a mixture thereof.

5. The pharmaceutical composition according to claim 4 wherein the alkali and alkaline earth metals are selected from sodium, potassium, magnesium and calcium.

6. The pharmaceutical composition according to claim 4 wherein the alkaline-reacting compound is magnesium carbonate.

7. The pharmaceutical composition according to any of claims 1-6 which is encapsulated.

8. The pharmaceutical composition according to any of claims 1-7 further comprising a lubricant.

9. The pharmaceutical composition according to claim 8 wherein the lubricant is selected from sodium stearylfumarate, magnesium stearate, hydrogenated vegetable oil, stearic acid, calcium stearate, glyceryl behenate, sodium lauryl sulphate and talc or a mixture thereof.

10. The pharmaceutical composition according to any of claims 1-9 wherein both pellets (a) and (b) comprise:
(1) an inert core;
(2) an active layer, deposited over said inert core (1), comprising lansoprazole or a pharmaceutically acceptable salt thereof, at least one binder preferably selected from hydroxypropylcellulose, hydroxypropylmethylcellulose, and sucrose or a combination thereof, and, in the case of pellets (b) additionally at least one alkaline-reacting compound;
(3) a separating layer; and
(4) an enteric layer over the separating layer (3).

11. The pharmaceutical composition according to any of claims 1-10 wherein pellets (a) comprise:
(1) an inert core;
(2) an active layer, deposited over said inert core (1), comprising lansoprazole or a pharmaceutically acceptable salt thereof, and hydroxypropylmethylcellulose;
(3) a separating layer comprising hydroxypropylmethylcellulose; and
(4) an enteric layer over the separating layer (3).

12. The pharmaceutical composition according to any of claims 1-11 wherein pellets (b) comprise:
(1) an inert core;
(2) an active layer, deposited over said inert core (1), comprising lansoprazole or a pharmaceutically acceptable salt thereof, hydroxypropylcellulose, corn starch, sucrose, low substituted hydroxypropylcellulose and magnesium carbonate;
(3) a separating layer comprising sucrose, corn starch, low substituted hydroxypropylcellulose, hydroxypropylcellulose; and
(4) an enteric layer over the separating layer (3).

13. A capsule comprising the pharmaceutical composition according to any of claims 1-6 together with a lubricant.

14. The capsule according to claim 13 wherein:
pellets (a) comprise:
(1) an inert core;
(2) an active layer, deposited over said inert core (1), comprising lansoprazole or a pharmaceutically acceptable salt thereof, and hydroxypropylmethylcellulose,
(3) a separating layer comprising hydroxypropylmethylcellulose; and
(4) an enteric layer over the separating layer (3);
and pellets (b) comprise:
(1) an inert core;
(2) an active layer, deposited over said inert core (1), comprising lansoprazole or a pharmaceutically acceptable salt thereof, corn starch, sucrose, low substituted hydroxypropylcellulose, hydroxypropylcellulose and magnesium carbonate;
(3) a separating layer comprising sucrose, corn starch, low substituted hydroxypropylcellulose, hydroxypropylcellulose; and
(4) an enteric layer over the separating layer (3).

15. A pharmaceutical composition according to any of claims 1-12 for use in the treatment and/or prophylaxis of ulcers of the stomach and duodenum, and NSAID-induced ulcers; gastroesophageal reflux disease (GERD); severe erosive esophagitis; poorly responsive systematic GERD; pathological hypersecretory conditions such as Zollinger-Ellison Syndrome; and adjunctive treatment of *Helicobacter pylori* infection, alongside antibiotics; or combinations of any of the above disorders.
